# EUROPEAN PATENT SPECIFICATION

(11) **EP 4 387 559 B1**
(45) Date of publication and mention of the grant of the patent: **20.08.2025**
(21) Application number: 23719777.7
(22) Date of filing: 19.04.2023
(51) Int. Cl.: A61B 17/00, A61B 90/00, A61B 8/08

(54) **FLEXIBLE GRADUATED HOOK-WIRE AND KIT FOR POSITIONING THEREOF IN A PATIENT'S BREAST, AXILLA OR OTHER SOFT TISSUE**
FLEXIBLER ABGESTUFTER HAKENDRAHT UND KIT ZUR POSITIONIERUNG DAVON IN DER BRUST, AXILLA ODER ANDEREM WEICHGEWEBE EINES PATIENTEN
FIL-CROCHET GRADUÉ FLEXIBLE ET KIT DE POSITIONNEMENT DE CELUI-CI DANS LE SEIN, L'AISSELLE OU UN AUTRE TISSU MOU D'UNE PATIENTE

(30) Priority: 19.04.2022 LU 501863
(43) Date of publication of application: 26.06.2024
(73) Proprietor: Cheretakis, Alexandre, 1245 Collonge-Bellerive (CH)
(72) Inventor: Cheretakis, Alexandre, 1245 Collonge-Bellerive (CH)
(74) Representative: Ipsilon Benelux
(86) International application number: PCT/EP2023/060057
(87) International publication number: WO 2023/203044

(56) References cited:
- WO-A1-2021/217007
- WO-A1-2021/222340
- US-A- 5 749 887
- US-A1- 2019 388 070

## Description

### Technical field

The invention relates to the field of targeting tissue areas of interest. In particular, the present invention relates to the field of targeting a breast cancerous tissue or lymph node or other soft tissue for removal.

### Background art

Today, the prognosis of breast cancer, which is a major threat and concern to women, has improved significantly over the past few years. Indeed, the improvement of early detection by mammographic imaging increases the rate of discovery of early-stage invasive tumors and precancerous lesions of the in-situ type that are generally non-palpable lesions.

After radiological detection, a conservative surgery can be performed, which consists of removing only the tumor, as opposed to the empirical mastectomy where the entire breast gland is removed.

What has also contributed to the improvement of the prognosis of breast cancer, is the progress of systematic treatments such as neoadjuvant chemotherapies that are administered before surgery. The goal of this therapeutic approach is to reduce the size of the tumor before surgery and even, in some cases, to obtain a radiological disappearance of the tumor.

In this respect, it is known in prior art to use a hook, which acts as a marker that is placed by radiologists before any chemotherapy treatment, to find the tumor location and also in case there is a disappearance of the tumor after a neoadjuvant treatment.

The large number of non-palpable tumors now identified by radiology has led to an increase in the number of surgeries requiring pre-surgical tumor localization using hooks.

For that matter, non-palpable tumors are targeted before surgery by a hook placement, which will be done either under ultrasound imaging if visible, or by stereotactic location, by mammography or even by MRI "Magnetic Resonance Imaging" which is more rarely performed in this case.

In the case of ultrasound-guided targeting, the radiologist will harpoon the tumor through an entry point that he will choose on the skin of the patient's breast and let the outer end of the hook's wire outside the entry point. After said targeting, a surgeon will perform the lumpectomy technique, which in a conservative treatment consisting of following the hook from its entry point, estimating the distance from the skin to the tumor, surgically dissecting along the hook to its tip and finally removing the tumor.

However, even if the radiologist gives a length between the hook and the skin entry point, during the operation, the surgeon has to peel off the skin, which deforms the breast. Thus, causing a disruption of the distance given by the radiologist.

Added to that, especially when using the hooks of prior art, it has been observed that once the skin is reclined (during the surgery), the direction of the cable is no longer the same as before opening the skin, that is because the position of the patient is changed with respect to the radiological examination and the breast's position is also changed. Therefore, it is very difficult to know the exact distance to the tumor.

This difficulty increases the volume of the removed gland, which consequently increases the risk of a large dissected tissue volume by exceeding the tumor's position, or even worse, the risk of an incomplete excision can also increase, thus causing an additional surgery.

In the same way, breast cancer can disseminate by lymphatic way in the lymph nodes, i.e. can also make axillary metastases. The preoperative location of the affected lymph node is also determined by placing a hook under ultrasound imaging. Unfortunately, the tissue of the axillary cavity is essentially made up of fat, a tissue in which the current hooks, being mainly of two-dimensional configuration, have difficulty in anchoring themselves without moving right afterwards.

In case of a hook displacement, even minimal of a few millimeters, the surgeon may be unable to find the lymph node, which will constitute a failure of the technique and will therefore impose a complete axillary curage (complete removal), the latter causing a much higher morbidity to the patient.

Patent document US 6,261,240 B1 discloses a sentinel lymph node localization wire having a distal end comprising an anchor configured to be placed at the location of the targeted cancer cells.

Patent document published US 2004/0077971 A1 discloses a needle used to mark a tissue area in a breast, the needle includes two hooks at its distal end that are slidable and deployed to anchor said needle, the needle further includes markers on its rigid tube to provide depth information.

Patent document US 5,749,887 B1 discloses a hook-wire for localization of a tumor in a breast, according to the preamble of claim 1.

Patent document published WO 2021/217007 A1 discloses tissue marking devices and methods that enable marking a target tissue more than 24 hours prior to surgery.

Patent document published WO 2021/222340 A1 discloses a biopsy marker that can emit near infrared fluorescence for location of a biopsy site.

Patent document published US 2019/388070 A1 discloses an excision device including a guide rod assembly and methods of operating the same.

Solutions of prior art have room for improvement, notably to avoid the commonly occurred displacements in the patient's breasts as well as avoiding the non-accuracy of cancerous tissues targeting.

### Summary of invention

The invention relates to a hook-wire for localization of a tumor in a breast and is defined by the features of independent claim 1. Preferred embodiments are given in the dependent claims.

### Technical problem

The present invention addresses the above-mentioned deficiencies and aims at providing a hook-wire allowing breast surgeons to know in a reliable way the exact distance to the tumor.

Overall, the invention allows achieving a smaller resected volume and a more satisfactory aesthetic result for the patient's breast post-surgery.

### Solution

The above-stated problem is solved by a hook-wire for localization of a tumor in a breast, comprising: an elongate member with a proximal end and a distal end; at least one hook at the distal end of the elongate member, configured for insertion in a constrained state into a needle and expansion when exiting said needle in order to anchor the tumor in said breast; wherein the elongate member and the at least one hook are formed by at least two metal wires which are twisted around each other continuously from the proximal end to the distal end and terminating each at said distal end with a free curved portion forming one of the at least one hook, wherein the hook-wire further comprises a sheathing on the elongate member, forming a reading surface with indicia, said reading surface extending over at least 70% of a length of the elongate member between the proximal end and the distal end.

Preferably, the at least two wires are flexible, i.e. can bend and/or fold easily along a total length of the hook-wire, and respect hygiene standards for operating room instrumentation and medical devices, and are preferably made from a non-magnetic material such as stainless steel or titanium.

Preferably, the at least two metal wires twisted around each other continuously is with a constant pitch over at least 70% of a length of the elongate member between the proximal end and the distal end, more preferably from the proximal end to the distal end.

According to a preferred embodiment, the at least two metal wires are three of said metal wires and the at least one hook corresponding to three of said hooks.

According to a preferred embodiment, the at least two hooks, in the expended state, are evenly angularly distributed around a main axis of the elongate member.

According to a preferred embodiment, the free curved portion of each of the at least two hooks forms, from the distal end of the elongate member to a free end of said hook, a quarter of circle.

According to a preferred embodiment, the quarter of circle shows a radius comprised between 7 and 11 mm.

According to a preferred embodiment, the free end of each hook is at a distance from a main axis of the elongate member that is comprised between 7 and 11 mm.

According to a preferred embodiment, each of the at least two hooks has a free end portion perpendicular to the main axis of the elongate member or inclined relative to said axis by not more than 20°.

According to a preferred embodiment, each of the at least two metal wires has a diameter comprised between 0.015 and 0.03 mm.

Preferably the sheathing is thin and being made of a deformable material, and more preferably made of graduated silicone.

According to a preferred embodiment, the indicia are distributed along the elongate member at fixed interval, preferably of 10 mm.

According to a preferred embodiment, the indicia are colored with a different color after each two intervals.

According to a preferred embodiment, the indicia further comprise Arabic numbers distributed over the colored indicia.

According to a preferred embodiment, the indicia comprise Arabic numbers.

Preferably the indicia are engraved depth indicators on the sheathing, and the latter comprises 24 indicia.

The invention also relates to a kit for localization of a tumor in a breast, comprising: a needle; a guide element being configured to slide coaxially inside the needle; the hook-wire of the invention, wherein said hook-wire being pre-inserted inside the guide element; and a ruler comprising identical indicia as of the hook-wire.

According to a preferred embodiment, the hook of the hook-wire being in a constrained position and inside the guide element.

According to a preferred embodiment, the ruler is made of a sterile medical paper comprising a length of 120 mm and width of 20 mm.

According to a preferred embodiment, the kit being a sterile single-use kit.

Advantageously the hook-wire, and more particularly the elongate member is flexible, which is achieved by the flexibility of the at least two wires along with the flexible sheathing, said flexibility allows an easy manipulation of the hook-wire.

The twisted wires will create a shadow during an ultrasound check, which will advantageously allow the surgeon to follow the hook-wire's path, therefore enabling improved targeting of the tumor.

Moreover, the flexibility of the hook-wire enhances the retention of the position of the anchor nearby a tumor by minimizing the transmission of eventual parasite efforts to the distal end. In fact, said parasite efforts can occur following a change of a patient's position while having the hook-wire anchored in its breast.

Another advantage of the invention, is the increased anchoring due to the hook being comprised of three anchors that further guarantee a safe fastening of the hook wire into the breast tissue, and the combination with colored and numbered indicia on the sheathing, the reading of indicia and determination of depth is significantly improved, therefore allowing the surgeon to precisely localize the tumor, which more advantageously results into a volume of dissected breast tissue that is as small as possible.

### Brief description of the drawings

Figure 1 is an illustration of a side view of three twisted metal wires of a hook-wire;
Figure 2 shows a representation of a graduated sheathing configured to wrap an elongate member of the hook-wire;
Figure 3 depicts a side view of the graduated hook-wire according to the invention;
Figure 4 shows a side view illustration of a needle;
Figure 5 shows a side view illustration of a guide element;
Figure 6 is a schematic representation of a patient's breast comprising a tumor being localized during a mammography and using the graduated hook-wire of figure 3.
Figure 7 shows a ruler configured to be used along with the hook-wire of the invention.

### Detailed description of the drawings

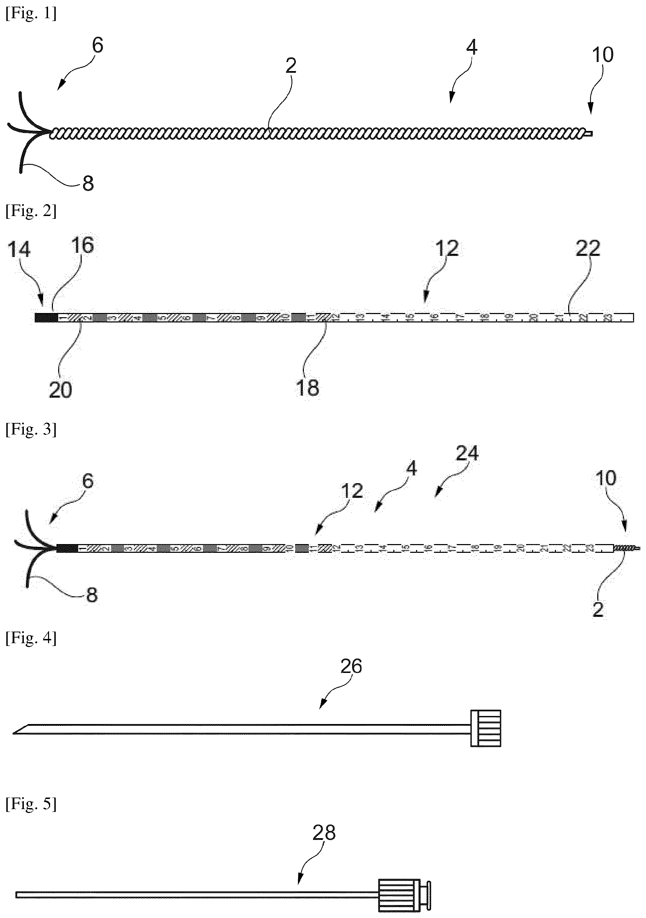

Figure 1 is an illustration of a side view of at least two metal wires 2 of a hook-wire configured to be positioned inside a patient breast and nearby a tumor or a sentinel lymph node.

More precisely, figure 1 depicts three twisted metal wires 2 of an elongate member 4, the latter further comprises an anchoring region 6 at its distal end, which is made of at least one hook 8. Preferably, the at least one hook 8 corresponds to three of said hooks 8.

In fact, the anchoring region 6 and the elongate member 4, are made of the three metal wires 2 which are twisted around each other in a continuous manner from a proximal end 10 to the distal end comprising the hooks 8.

The metal wires 2 are twisted around each other preferably with a constant pitch over at least 70%, more preferably over the whole lengths of the elongate member 4.

Preferably, each wire 2 comprises a diameter of 0.015 mm and above and/or a diameter of 0.03 mm or below, and more preferably, each wire 2 comprises a diameter of 0.02 mm.

The proximal end 10 is configured to stay outside the patient's breast while the anchoring region 8 is configured to anchor the tumor in the breast. For that matter, each hook 8 is formed by a free curved portion of each one of the three wires 2, and the proximal end 10 can comprise the wires 2 for instance in a twisted state or in an untwisted state.

Each hook 8 of figure 1 is illustrated in an expanded state subsequent to a constrained state which will be further explained later on in the present description.

In this configuration, the hooks 8 are evenly angularly distributed around a main axis of the elongate member 4, and preferably, each hook 8 is separated from another hook 8 by a 120° angle.

Preferably, each hook 8 have a quarter of a circle shape, or a substantially C shaped form, starting from the distal end of the elongate member to a free end of said hook 8. In this configuration, the free end portion can be perpendicular to the main axis of the elongate member 4 or inclined relative to said axis by preferably ±10°, and more preferably not more than 20°.

Contrary to hook systems configured to perform ablation of tissues wherein said hook systems require a further inclination angle, the main purpose of hooks 8 of the anchoring region 6 is to anchor the hook-wire and to maintain the position of the latter is a secure way in the breast, therefore, there is no need to exceed the 20° inclination of the free end of each hook 8 relative to the main axis of the elongate member 4.

The quarter of a circle shape of each hook 8 can be identified by a radius of the circle being comprised between 7 mm and 11 mm, and preferably a radius of 9 mm. In this configuration, the radius of each hook 8 is equal to the distance between the free end portion of said hook 8 and the distal end of the elongate member 4.

The distribution of the hooks combined with their C shaped form according to the invention enables to sufficiently secure the hook-wire's position in the breast's cancerous tissues and maintain the position even when movements of the breast occur posteriorly.

Advantageously, the twisted wires enable on the one hand to create a visual path that can be visualized during a pre-surgery mammography by the surgeon, and on the other hand, the twisting can make the manufacturing of the hooks easy. In fact, a triangular arrangement of the wires 2 before twisting, can for instance allow to easily reach the homogeneous distribution at a 120° angle of the hooks 8.

Figure 2 shows a representation of a graduated sheathing configured to wrap the elongate member 4 of figure 1.

In reference to figure 2, the sheathing 12 forms a reading surface with indicia that are preferably distributed along the total length of the sheathing 12 at fixed interval of preferably 10 mm for each interval.

The reading surface of the sheathing 12 can extend at 360° around the total circumference of said sheathing 12.

Advantageously, the indicia are colored with a different color after each two intervals. Preferably, indicia from a first interval 16 starting from a distal end 14 of the sheathing 12 to a twelfth interval 18, are colored. Therefore, the first 12 cm of the sheath are colored.

For that matter, the first interval 16 can be black, followed by a second interval 20 comprising a black hatch pattern, and the twelfth interval 18 can comprise a red hatch pattern for example.

The indicia can comprise visible Arabic numbers distributed and incremented along the length of the elongate member 4, preferably with an Arabic number on each interval 20, the numbers are visible on the surface of the sheathing.

The colored indicia of the sheathing 12 and the Arabic numbering advantageously enable fast and easy reading of the intervals, thus, enabling quick determination of the depth of the tumor from the breast's skin by the surgeon during the surgery.

Preferably, the sheathing 12 is flexible and respect hygiene standards for operating room instrumentation usage and medical devices, and made from silicone that is suitable for such usage, for instance, it can be platinum-catalyzed silicone or peroxide-catalyzed silicone. The sheathing 12 is advantageously continuous over its whole length.

Indicia of the reading surface can be made in several ways, such as using a heat shrink tube, or by medical plastic coating, or by laser coloring directly on an outer surface of each interval.

Figure 3 depicts a side view of the graduated hook-wire 24 according to the invention, wherein the sheathing 12 of figure 2 wraps the elongate member 4 of figure 1.

In reference to figure 3, the graduated sheathing 12 extends over at least 70%, and preferably over 80% of a length of the elongate member 4 between the proximal end 10 and the distal end.

Preferably, the total length of the reading surface is 24 cm while the total length of the elongate member 4 is for example 30 cm.

The graduated hook-wire 24 is comprised in a kit for localization of a tumor in the patient's breast, the kit further comprises a needle and a guide element.

Preferably, the kit is a sterile single-use kit which is a medical device, and all of its components respect the standards: ISO 11 607-1 and ISO 11 607-2.

Figures 4 and 5 show a side view illustration of the needle and the guide element, respectively.

In reference to both figures 4 and 5, the needle 26 is a hollow needle 26 configured be inserted into the breast beforehand to insertion of the guide element 28 in its hollow. For that matter, the guide element 28 is configured to slide coaxially inside the needle 26. During mammography ultrasonography, or RMI, the slide movement can be followed by an expansion of the anchoring region of the hook-wire outside the needle 26 and nearby its distal end.

Preferably, both the needle 26 and the guide element 28 comprise an overall length being 60 mm or above and/or being 150 mm or below, and can also be for instance 90 mm or 120 mm.

The needle 26 can comprise an outer surface of having indicia or being composed of an outer movable shell having indicia and helping with achieving a pre-measurement of the depth to the tumor in the breast during the pre-surgery mammography or ultrasonography.

The anchoring region 6 along with the elongate member 4 of the hook-wire 24 of figure 3 are configured to be inside the guide element 28 of the kit. In this configuration, each hook of the hook-wire is in a constrained position inside the guide element 28.

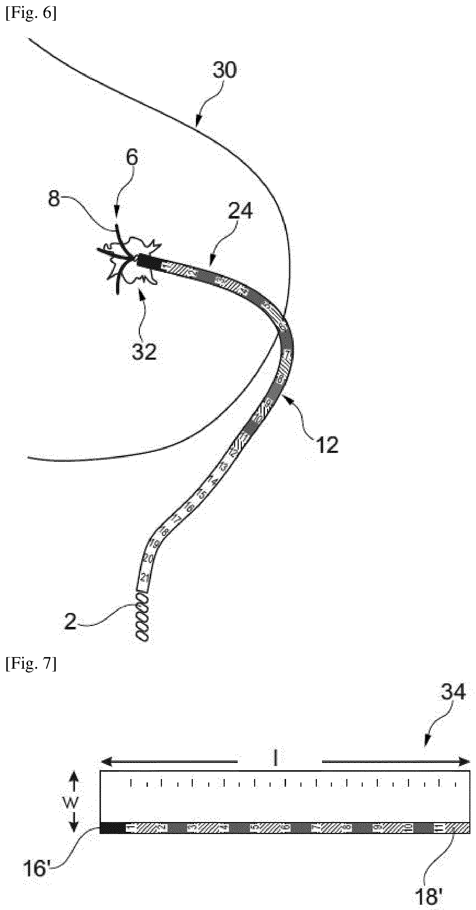

Figure 6 is a schematic representation of a patient's breast 30 comprising a tumor 32 being localized during a mammography and using the hook-wire 24 of figure 3.

It can be seen from figure 6 that the hook-wire 24 is flexible enabling its easy manipulation. The flexibility can be defined by the bending capability of the hook-wire along its total length, for instance, the elongate member of the hook-wire nearby the anchoring region 6, can be bent until forming a bending radius of preferably at least 10 mm and can still elastically return into its initial unbent state.

Each hook 8 of the anchoring region was beforehand in a constrained position in the guide element 28 of figure 5. In fact, the hooks 8 are movable through spring action from the constrained position into an anchoring position, the latter is the one depicted in figure 6.

Figure 7 shows a ruler 34 (graduated scale) configured to be used along with the hook-wire of the invention.

The hook-wire of figure the present invention is comprised in the kit for localization of a tumor in the patient's breast, the kit comprises the needle and the guide element, said kit further comprises a ruler 34 comprising identical indicia as of the sheathing of the hook-wire, and more precisely, identical to the colored indicia from the first interval 16 to the twelfth interval 18 of figure 2.

For that matter, the ruler 34 comprises twelve intervals of 1 cm each, from the first interval 16' to twelfth interval 18', and having identical colors to the one of the corresponding interval of the sheathing 12 of figure 2.

The different patterns and changing shades of gray in figure 7 are only to be understood as being different colors for each interval from the first interval 16' to the twelfth interval 18'. For instance, the first interval 16' is preferably black and the twelfth interval 18' can be red, similarly to the first 16 and twelfth interval 18 of figure 2.

Moreover, the ruler is made of a sterile medical paper comprising a length I of 120 mm and width w of 20 mm. The ruler enables the surgeon to verify and perform measurements parallelly to the hook-wire during the operation.

Advantageously, the colored indicia of the ruler 34 help the surgeon to quickly and easily perform the measure in order to effectively determine the depth of the tumor in the patient's breast.

Preferably, the ruler 34 is a medical sterile single-use kit respecting the standards: ISO 11 607-1 and ISO 11 607-2.

It is to note that the present disclosure is not limited only to the illustrated hook-wire. In fact, another aspect of the invention can be the use of the ruler along with a needle or any other medical element penetrating the human body and which is preferably sterile. It is preferable that said medical element comprises a small diameter, i.e. being less than 5 mm, and further comprises indicia with colored intervals on its outer surface.

In an advantageous manner, utilizing a sterile ruler during a surgery along with the medical element having similar colored indicia as on the ruler, will help the surgeon perform depth measurements easily and in a more effective way, notably when said medical element is subject to contamination by elements of the human body such as blood for instance.

## Claims

1. A hook-wire (24) for localization of a tumor (32) in a breast (30), comprising:
- an elongate member (4) with a proximal end (10) and a distal end; and
- at least one hook (8) at the distal end of the elongate member (4), configured for insertion in a constrained state into a needle (26) and expansion when exiting said needle (26) in order to anchor the tumor (32) in said breast (30);
wherein the elongate member (4) and the at least one hook (8) are formed by at least two metal wires (2) which are twisted around each other continuously from the proximal end (10) to the distal end and terminating each at said distal end with a free curved portion forming one of the at least one hook (8)
**characterized in that** the hook-wire (24) further comprises:
- a sheathing (12) on the elongate member (4), forming a reading surface with indicia, said reading surface extending over at least 70% of a length of the elongate member (4) between the proximal end (10) and the distal end.

2. The hook-wire (24) of claim 1, wherein the at least two metal wires (2) are three of said metal wires (2) and the at least one hook (8) corresponding to three of said hooks (8).

3. The hook-wire (24) of one of claims 1 and 2, wherein the at least two hooks (8), in the expanded state, are evenly angularly distributed around the main axis of the elongate member (4).

4. The hook-wire (24) of any one of claims 1 to 3, wherein the free curved portion of each of the at least two hooks (8) forms, from the distal end of the elongate member (4) to a free end of said hook (8), a quarter of a circle.

5. The hook-wire (24) of claim 4, wherein the quarter of a circle shows a radius comprised between 7 and 11 mm.

6. The hook-wire (24) of one of claims 4 and 5, wherein the free end of each hook (8) is at a distance from the main axis of the elongate member (4) that is comprised between 7 and 11 mm.

7. The hook-wire (24) of any one of claims 1 to 6, wherein each of the at least two hooks (8) has a free end portion perpendicular to the main axis of the elongate member (4) or inclined relative to said axis by not more than 20°.

8. The hook-wire (24) of any one of claims 1 to 7, wherein each of the at least two metal wires (2) has a diameter comprised between 0.015 and 0.03 mm.

9. The hook-wire (24) of any one of claims 1 to 8, wherein the indicia are distributed along the elongate member (4) at fixed interval, preferably of 10 mm.

10. The hook-wire (24) of claim 9, wherein the indicia are colored with a different color after each two intervals.

11. The hook-wire (24) of claim 10, wherein the indicia further comprise Arabic numbers distributed over the colored indicia.

12. The hook-wire (24) of any one of claims 1 to 11, wherein the indicia comprise Arabic numbers.

13. A kit for localization of a tumor (32) in a breast (30), comprising:
a needle (26);
a guide element (28) being configured to slide coaxially inside the needle (26);
the hook-wire (24) in accordance with any of claims 1 to 12, wherein said hook-wire (24) being pre-inserted inside the guide element (28); and
a ruler (34) comprising identical indicia as of the hook-wire (24).

14. The kit according to claim 13, wherein the hook (8) of the hook-wire (24) being in a constrained position and inside the guide element (28).

15. The kit according to any of claims 13 or 14, wherein the ruler (34) is made of a sterile medical paper comprising a length of 120 mm and width of 20 mm.

## Patentansprüche

1. Ein Hakendraht (24) zur Lokalisierung eines Tumors (32) in einer Brust (30), umfassend:
- ein längliches Element (4) mit einem proximalen Ende (10) und einem distalen Ende; und
- mindestens einen Haken (8) am distalen Ende des länglichen Elements (4), konfiguriert zur Einführung in einem eingeschränkten Zustand in eine Nadel (26) und zur Expansion beim Austritt aus der genannten Nadel (26), um den Tumor (32) in der genannten Brust (30) zu verankern; wobei das längliche Element (4) und der mindestens eine Haken (8) durch mindestens zwei Metalldrähte (2) gebildet wird, die sich kontinuierlich vom proximalen Ende (10) bis zum distalen Ende umeinander drehen und jeweils am genannten distalen Ende mit einem freien gebogenen Abschnitt enden, der einen der mindestens einen Haken (8) bildet:
**dadurch gekennzeichnet, dass** der Hakendraht (24) weiter umfasst:
- eine Ummantelung (12) auf dem länglichen Element (4), die eine Lesefläche mit Markierungen bildet, wobei sich die genannte Lesefläche über mindestens 70% der Länge des länglichen Elements (4) zwischen dem proximalen Ende (10) und dem distalen Ende erstreckt.

2. Der Hakendraht (24) nach Anspruch 1, wobei die mindestens zwei Metalldrähte (2) drei der genannten Metalldrähte (2) sind und der mindestens eine Haken (8) drei der genannten Haken (8) entspricht.

3. Der Hakendraht (24) nach einem der Ansprüche 1 und 2, wobei die mindestens zwei Haken (8) im expandierten Zustand gleichmäßig um die Hauptachse des länglichen Elements (4) verteilt sind.

4. Der Hakendraht (24) nach einem der Ansprüche 1 bis 3, wobei der freie gebogene Abschnitt jedes der mindestens zwei Haken (8) vom distalen Ende des länglichen Elements (4) bis zu einem freien Ende des genannten Hakens (8) ein Viertel eines Kreises bildet.

5. Der Hakendraht (24) nach Anspruch 4, wobei das Viertel eines Kreises einen Radius zwischen 7 und 11 mm aufweist.

6. Der Hakendraht (24) nach einem der Ansprüche 4 und 5, wobei das freie Ende jedes Hakens (8) in einem Abstand von der Hauptachse des länglichen Elements (4) liegt, der zwischen 7 und 11 mm beträgt.

7. Der Hakendraht (24) nach einem der Ansprüche 1 bis 6, wobei jeder der mindestens zwei Haken (8) einen freien Endabschnitt senkrecht zur Hauptachse des länglichen Elements (4) oder relativ zu der genannten Achse um nicht mehr als 20° geneigt hat.

8. Der Hakendraht (24) nach einem der Ansprüche 1 bis 7, wobei jeder der mindestens zwei Metalldrähte (2) einen Durchmesser zwischen 0.015 und 0.03 mm hat.

9. Der Hakendraht (24) nach einem der Ansprüche 1 bis 8, wobei die Markierungen entlang des länglichen Elements (4) in festen Abständen verteilt sind, vorzugsweise von 10 mm.

10. Der Hakendraht (24) nach Anspruch 9, wobei die Markierungen nach jeweils zwei Abständen mit einer anderen Farbe gefärbt sind.

11. Der Hakendraht (24) nach Anspruch 10, wobei die Markierungen weiterhin arabische Zahlen über den farbigen Markierungen umfassen.

12. Der Hakendraht (24) nach einem der Ansprüche 1 bis 11, wobei die Markierungen arabische Zahlen umfassen.

13. Ein Kit zur Lokalisierung eines Tumors (32) in einer Brust (30), umfassend:
eine Nadel (26);
ein Führungselement (28), konfiguriert zum koaxialen Gleiten innerhalb der Nadel (26);
der Hakendraht (24) gemäß einem der Ansprüche 1 bis 12, wobei der genannte Hakendraht (24) vorab innerhalb des Führungselements (28) eingeführt ist; und ein Lineal (34), das identische Markierungen wie der Hakendraht (24) umfasst.

14. Das Kit gemäß Anspruch 13, wobei der Haken (8) des Hakendrahts (24) in einer eingeschränkten Position und innerhalb des Führungselements (28) ist.

15. Das Kit gemäß einem der Ansprüche 13 oder 14, wobei das Lineal (34) aus sterilem medizinischem Papier besteht und eine Länge von 120 mm und eine Breite von 20 mm aufweist.

## Revendications

1. Un fil-crochet (24) pour la localisation d'une tumeur (32) dans un sein (30), comprenant :
- un élément allongé (4) avec une extrémité proximale (10) et une extrémité distale ; et
- au moins un crochet (8) à l'extrémité distale de l'élément allongé (4), configuré pour être inséré dans un état contraint dans une aiguille (26) et se déployer en sortant de ladite aiguille (26) afin d'ancrer la tumeur (32) dans ledit sein (30) ; dans lequel l'élément allongé (4) et le ou les crochets (8) sont formés par au moins deux fils métalliques (2) qui sont torsadés l'un autour de l'autre en continu de l'extrémité proximale (10) à l'extrémité distale et se terminant chacun à ladite extrémité distale par une portion courbée libre formant l'un desdits crochets (8) :
**caractérisé en ce que** le fil-crochet (24) comprend en outre :
- un gainage (12) sur l'élément allongé (4), formant une surface de lecture avec des indicateurs, ladite surface de lecture s'étendant sur au moins 70 % de la longueur de l'élément allongé (4) entre l'extrémité proximale (10) et l'extrémité distale.

2. Le fil-crochet (24) selon la revendication 1, dans lequel les au moins deux fils métalliques (2) sont trois desdits fils métalliques (2) et le ou les crochets (8) correspondant à trois desdits crochets (8).

3. Le fil-crochet (24) selon l'une des revendications 1 et 2, dans lequel les au moins deux crochets (8), dans l'état déployé, sont répartis de manière angulaire uniforme autour de l'axe principal de l'élément allongé (4).

4. Le fil-crochet (24) selon l'une des revendications 1 à 3, dans lequel la portion courbée libre de chacun des au moins deux crochets (8) forme, de l'extrémité distale de l'élément allongé (4) à une extrémité libre dudit crochet (8), un quart de cercle.

5. Le fil-crochet (24) selon la revendication 4, dans lequel le quart de cercle présente un rayon compris entre 7 et 11 mm.

6. Le fil-crochet (24) selon l'une des revendications 4 et 5, dans lequel l'extrémité libre de chaque crochet (8) est à une distance de l'axe principal de l'élément allongé (4) comprise entre 7 et 11 mm.

7. Le fil-crochet (24) selon l'une des revendications 1 à 6, dans lequel chacun des au moins deux crochets (8) a une portion d'extrémité libre perpendiculaire à l'axe principal de l'élément allongé (4) ou inclinée par rapport audit axe de pas plus de 20°.

8. Le fil-crochet (24) selon l'une des revendications 1 à 7, dans lequel chacun des au moins deux fils métalliques (2) a un diamètre compris entre 0,015 et 0,03 mm.

9. Le fil-crochet (24) selon l'une des revendications 1 à 8, dans lequel les indicateurs sont répartis le long de l'élément allongé (4) à intervalle fixe, de préférence de 10 mm.

10. Le fil-crochet (24) selon la revendication 9, dans lequel les indicateurs sont colorés avec une couleur différente après chaque deux intervalles.

11. Le fil-crochet (24) selon la revendication 10, dans lequel les indicateurs comprennent en outre des chiffres arabes répartis sur les indicateurs colorés.

12. Le fil-crochet (24) selon l'une des revendications 1 à 11, dans lequel les indicateurs comprennent des chiffres arabes.

13. Un kit pour la localisation d'une tumeur (32) dans un sein (30), comprenant :
une aiguille (26);
un élément de guidage (28) étant configuré pour glisser coaxialement à l'intérieur de l'aiguille (26);
le fil-crochet (24) selon l'une quelconque des revendications 1 à 12, dans lequel ledit fil-crochet (24) est pré-inséré à l'intérieur de l'élément de guidage (28); et une règle (34) comprenant des indicateurs identiques à ceux du fil-crochet (24).

14. Le kit selon la revendication 13, dans lequel le crochet (8) du fil-crochet (24) est dans une position contrainte et à l'intérieur de l'élément de guidage (28).

15. Le kit selon l'une quelconque des revendications 13 ou 14, dans lequel la règle (34) est faite de papier médical stérile comprenant une longueur de 120 mm et une largeur de 20 mm.
